# EUROPÄISCHE PATENTANMELDUNG

(11) **EP 1 772 722 A1**
(43) Veröffentlichungstag der Anmeldung: **11.04.2007**
(21) Anmeldenummer: 05021964.1
(22) Anmeldetag: 07.10.2005
(51) Int. Cl.: G01N 15/14, B01L 3/00, C12M 3/00

(54) **Reaktionskammer**

(71) Anmelder: Micronas Holding GmbH, 79108 Freiburg i.Br. (DE); Universität Rostock, 18055 Rostock (DE)
(72) Erfinder: Baumann, Werner, Dr. rer. nat, 18055 Rostock (DE); Lehmann, Mirko, Dr. rer. nat, 79117 Freiburg (DE); Freund, Ingo, Dipl.-Ing. (FH), 79117 Freiburg (DE)
(74) Vertreter: Huwer, Andreas

(57) **Zusammenfassung**

Eine Reaktionskammer (1) weist eine Innenhöhlung (3) auf, die mindestens eine Einlassöffnung (4) und eine Auslassöffnung (5) für ein Fluid hat. Zum Erzeugen einer in der Innenhöhlung (3) von der Einlassöffnung (4) zu der Auslassöffnung (5) verlaufenden Fluid-Strömung ist eine Fördereinrichtung vorgesehen. Die Reaktionskammer (1) hat zumindest eine von der Fluid-Strömung durch Scherkräfte beaufschlagte Begrenzungswand (2), an der wenigstens ein Aufnahmebereich (6) für ein strömungsempfindliches Reaktionszentrum (7), wie z.B. eine an der Begrenzungswand (2) immobiliserte biologische Zelle, vorgesehen ist, das dazu geeignet ist, einen in dem Fluid enthaltenen Stoff aufzunehmen und/oder einen Stoff an das Fluid abzugeben. Die Begrenzungswand (2) weist mindestens ein Strömungshindernis für die Fluid-Strömung auf, das derart zu dem Aufnahmebereich (6) benachbart ist, dass die Geschwindigkeit der Fluid-Strömung in dem Aufnahmebereich (6) geringer ist als außerhalb des Aufnahmebereichs (6).

## Beschreibung

Die Erfindung betrifft eine Reaktionskammer mit einer Innenhöhlung, die mindestens eine Einlassöffnung und wenigstens eine Auslassöffnung für ein Fluid aufweist, mit einer Fördereinrichtung zum Erzeugen einer in der Innenhöhlung von der Einlassöffnung zu der Auslassöffnung verlaufenden Fluid-Strömung, wobei die Reaktionskammer zumindest eine von der Fluid-Strömung durch Scherkräfte beaufschlagte Begrenzungswand hat, an der wenigstens ein Aufnahmebereich für ein strömungsempfindliches Reaktionszentrum, wie z.B. eine an der Begrenzungswand immobiliserte biologische Zelle, vorgesehen ist, das dazu geeignet ist, einen in dem Fluid enthaltenen Stoff aufzunehmen und/oder einen Stoff an das Fluid abzugeben. Unter einem Fluid wird ein flüssiges oder gasförmiges Medium verstanden.

Eine derartige Reaktionskammer, bei der in dem Aufnahmebereich zu untersuchende biologische Nerven-Zellen angeordnet sind, die an der Oberfläche einer Begrenzungswand der Reaktionskammer immobilisiert sind, ist aus der Praxis bekannt. Die Innenhöhlung der Reaktionskammer wird mit Hilfe einer Fördereinrichtung mit einer Lösung durchströmt, die Nährstoffe für die Zellen enthält. Die Lösung dient außerdem für den Abtransport von Stoffwechselprodukten, welche die Zellen abgeben. Mit Hilfe der Reaktionskammer ist es möglich, die Zellen über längere Zeit am Leben zu erhalten, um Messungen an den Zellen durchzuführen. Zu diesem Zweck sind in die Begrenzungswand der Messkammer Aktionspotentialsensoren integriert, um elektrische Signale zu messen, die Rückschlüsse auf die Zellaktivität und die Kommunikation zwischen den Zellen ermöglichen. So kann beispielsweise die Reaktion der Zellen auf Wirkstoffe untersucht werden, die der Lösung beigemischt und dann den Zellen zusammen mit der Lösung zugeführt werden. Damit die Zellen am Leben erhalten werden können, muss die in der Innenhöhlung der Reaktionskammer befindliche Lösung kontinuierlich oder zumindest diskontinuierlich durch pulsierendes Zuführen frischer Lösung erneuert werden. Die dabei in der Reaktionskammer auftretende Strömung übt Scherkräfte auf die Zellen aus, welche die elektrischen Signale der Zellen beeinflussen. Die Zellen können sehr sensibel auf die Scherkräfte reagieren, wodurch die Messsignale gestört werden. Dabei kann es sogar vorkommen, dass die durch die Scherkräfte verursachten Störungen deutlich größer sind als das zu messende beispielsweise durch den Kontakt der Zellen mit dem Wirkstoff hervorgerufene Nutzsignal, so dass das Nutzsignal nicht oder nicht mit ausreichender Genauigkeit gemessen werden kann. Bei einer pulsierenden Zuführung der Lösung können außerdem während der Pulspausen Signalveränderungen an den Zellen beobachtet werden, die durch eine Ansäuerung der Umgebung der Zellen zurückzuführen sind. Auch dadurch wird die Messgenauigkeit beeinträchtigt.

Es besteht deshalb die Aufgabe, eine Reaktionskammer der eingangs genannten Art zu schaffen, die einen schnellen Stoffaustausch zwischen dem mindestens einen Reaktionszentrum und dem Fluid ermöglicht und bei der während des Förderns des Fluids nur geringe Scherkräfte an dem Reaktionszentrum auftreten.

Diese Aufgabe wird dadurch gelöst, dass die Begrenzungswand mindestens ein Strömungshindernis für die Fluid-Strömung aufweist, das derart zu dem Aufnahmebereich benachbart ist, dass die Geschwindigkeit der Fluid-Strömung in dem Aufnahmebereich geringer ist als außerhalb des Aufnahmebereichs.

In vorteilhafter Weise wird durch das Strömungshindernis in unmittelbarer Nähe der Oberfläche der Begrenzungswand in dem Aufnahmebereich für das Reaktionszentrum die Strömungsgeschwindigkeit des Fluids während des Förderns des Fluids reduziert, so dass nur eine sehr geringe Scherkraft auf ein beispielsweise an der Begrenzungswand immobilisiertes strömungsempfindliches Reaktionszentrum ausgeübt wird. Dennoch kann das Fluid in der Innenhöhlung der Reaktionskammer relativ schnell erneuert werden, da die Strömungsgeschwindigkeit des Fluids außerhalb des Wirkungsbereichs des Strömungshindernisses größer ist als in dem Aufnahmebereich. Somit steht in einem gewissen Abstand zu dem Reaktionszentrum stets frisches Fluid zur Verfügung, so dass durch Diffusion ein schneller Stoffaustausch zwischen dem Reaktionszentrum und diesem Fluid erfolgen kann. Bei der Entwicklung von Medikamenten ermöglicht die Reaktionskammer durch Zugabe einer Testsubstanz oder eines Teststoffs zu dem Fluid im Bereich des Reaktionszentrums eine schnelle Einstellung einer gewünschten Testsubstanz-Konzentration. Mit Hilfe der Reaktionskammer ist es außerdem möglich, bei einer chemischen Reaktion, die bei Anwesenheit eines Katalysators zwischen mindestens zwei Reaktionspartnern auftritt, einen Stoffaustausch zwischen einem in dem Aufnahmebereich befindlichen Katalysator und den in einem Fluid enthaltenen Reaktionspartnern vorzunehmen und damit die chemische Reaktion zu ermöglichen.

Bei einer vorteilhaften Ausführungsform der Erfindung weist das Strömungshindernis eine Vielzahl von an der Begrenzungswand vorstehenden Vorsprüngen auf, die in Hauptströmungsrichtung der Fluid-Strömung und/oder quer dazu voneinander beabstandet sind und den Aufnahmebereich zwischen sich einschließen. Dadurch ist es möglich, in einem relativ großen oberflächennahen Bereich der Begrenzungswand der Innenhöhlung eine Reduzierung der auf die Reaktionszentren einwirkenden Scherkräfte zu erreichen, so dass eine entsprechend große Anzahl von Reaktionszentren in der Reaktionskammer gleichzeitig untersucht werden kann.

Bei einer zweckmäßigen Ausgestaltung der Erfindung weisen die Vorsprünge unterschiedliche Querschnittsabmessungen auf Die Fluid-Strömung wird dann noch besser an dem Strömungshindernis gebrochen, so dass sich die Strömungsgeschwindigkeit in einem oberflächennahen Bereich der Begrenzungswand weiter reduziert.

Vorteilhaft ist, wenn das Strömungshindernis mindestens zwei sich quer zur Hauptströmungsrichtung der Fluid-Strömung erstreckende Reihen mit voneinander beabstandeten Vorsprüngen aufweist, wenn die Reihen in Hauptströmungsrichtung voneinander beabstandet sind, und wenn die Vorsprünge einer ersten Reihe auf Lücke zu den Vorsprüngen einer zweiten Reihe versetzt sind. Auch durch diese Maßnahme kann in einem oberflächennahen Bereich der Begrenzungswand ein hoher Strömungswiderstand und somit eine geringe Strömungsgeschwindigkeit erreicht werden.

Bei einer vorteilhaften Ausgestaltung der Erfindung sind die Abmessungen, welche die Vorsprünge quer zur Erstreckungsebene der Begrenzungswand aufweisen, größer als der lichte Abstand zwischen den Vorsprüngen. In einem oberflächennahen Bereich der Begrenzungswand ergibt sich dann zwischen den Vorsprüngen ein noch größerer Strömungswiderstand.

Bei einer bevorzugten Ausführungsform der Erfindung betragen (beträgt) die Abmessungen, welche die Vorsprünge quer zur Erstreckungsebene der Begrenzungswand aufweisen und/oder der lichte Abstand zwischen den Vorsprüngen zwischen 10 und 200 µm, insbesondere zwischen 20 und 100 µm, gegebenenfalls zwischen 30 und 70 µm und bevorzugt zwischen 40 und 60 µm. Mit diesen Abmessungen konnten in der Praxis die Scherkräfte, die durch die Fluid-Strömung auf in dem Aufnahmebereich angeordnete, an der Begrenzungswand immobilisierte biologische Zellen ausgeübt werden, deutlich reduziert werden.

Bei einer vorteilhaften Ausführungsform der Erfindung weisen der Fluid-Strömung zugewandte Seitenflächen der Vorsprünge eine konkave Form auf An den Seitenflächen ergibt sich dann ein besonders großer Strömungswiderstand.

Vorteilhaft ist, wenn die Vorsprünge als Säulen ausgestaltet sind, die mit ihrer Längserstreckung vorzugsweise etwa normal zur Erstreckungsebene der Begrenzungswand angeordnet sind. Die das Strömungshindernis aufweisende Begrenzungswand der Innenhöhlung kann dann mit Methoden der Mikrosystemtechnik und/oder der Halbleiterfertigung kostengünstig hergestellt werden, beispielsweise indem auf die Oberfläche eines Substrats eine die späteren Säulen abdeckende ätzbeständige Maske aufgebracht und das Substrat danach mit einem Ätzmittel in Kontakt gebracht wird, um die die zwischen den Säulen befindlichen Bereiche abzutragen und dadurch den Aufnahmebereich für das mindestens eine Reaktionszentrum zu bilden.

Die Säulen weisen vorzugsweise einen eckigen, vorzugsweise rechteckigen und/oder quadratischen Querschnitt auf Dadurch wird ein großer Strömungswiderstand des Strömungshindernisses ermöglicht. Außerdem lässt sich das Strömungshindernis kostengünstig herstellen.

Vorteilhaft ist, wenn mindestens zwei Säulen mit rechteckigem oder länglichem Querschnitt mit ihrer Breitseite in unterschiedliche, vorzugsweise rechtwinklig zueinander verlaufende Richtungen weisen. Dadurch kann für aus unterschiedlichen Richtungen auf das Strömungshindernis einströmende Fluid-Teilströme gleichermaßen ein hoher Strömungswiderstand des Strömungshindernisses erreicht werden.

Bei einer bevorzugten Ausführungsform der Erfindung sind (ist) mehrere vorzugsweise auf einer Kreislinie angeordnete Einlassöffnungen und/oder eine ringförmige Einlassöffnung um eine zentrale Auslassöffnung herum angeordnet, wobei der Aufnahmebereich und das Strömungshindernis zwischen den Einlassöffnungen (der Einlassöffnung) und der Auslassöffnung vorgesehen sind und diese vorzugsweise ringförmig umgrenzen. Dadurch wird über den Aufnahmebereich eine gleichmäßige Reduzierung der Strömungsgeschwindigkeit des Fluids ermöglicht.

Selbstverständlich kann dies auch dadurch erreicht werden, dass mehrere vorzugsweise auf einer Kreislinie angeordnete Auslassöffnungen und/oder eine ringförmige Auslassöffnung um eine zentrale Einlassöffnung herum angeordnet sind (ist), und dass der Aufnahmebereich und das Strömungshindernis zwischen den Auslassöffnungen (der Auslassöffnung) und der Einlassöffnung vorgesehen sind und diese vorzugsweise ringförmig umgrenzen. In diesem Fall verläuft also die Fluid-Strömung in einem parallel zu der Erstreckungsebene der das Strömungshindernis aufweisenden Begrenzungswand beabstandeten Bereich der Innenhöhlung etwa radial von der Mitte nach außen.

Bei einer bevorzugten Ausführungsform der Erfindung ist in die Begrenzungswand mindestens ein Sensor zur Durchführung von Messungen an dem Reaktionszentrum integriert, wobei vorzugsweise mehrere Sensoren zur Messung unterschiedlicher Parameter, insbesondere des Aktionspotentials und/oder mindestens eines metabolischen Parameters einer biologischen Zelle nebeneinander angeordnet sind, vorzugsweise matrixförmig in mehreren Reihen und Spalten. Dabei ist der mindestens eine Sensor bevorzugt auf einem Halbleitersubstrat angeordnet, das einen Teilbereich der Begrenzungswand bildet. Der Sensor kann insbesondere einen ionenselektiven Feldeffekttransistor (ISFET) und/oder einen Digitalkondensator aufweisen. Gegebenenfalls ist es sogar möglich, dass in dem Aufnahmebereich ein Porationswerkzeug zum Einbringen einer Öffnung in die Zellmembran einer biologischen Zelle vorgesehen ist. Durch die Öffnung hindurch können dann Messungen und/oder Manipulationen an der Zelle vorgenommen werden.

Die Säulen sind zwischen den Sensoren vorzugsweise matrixförmig in mehreren Reihen und Spalten nebeneinander angeordnet Der Sensor ermöglicht dann ein einfaches Layout mit in parallel zueinander verlaufenden Reihen und Spalten angeordneten Sensorelementen. In einem um den die matrixförmig angeordneten Säulen aufweitenden Bereich der Begrenzungswand angeordneten ringförmigen Wandungsbereich der Begrenzungswand sind vorzugsweise weitere Säulen vorgesehen, deren Anordnung von einer Matrixform abweicht.

Nachfolgend ist ein Ausführungsbeispiel der Erfindung anhand der Zeichnung näher erläutert. Es zeigen:
- Fig. 1: einen Querschnitt durch eine Reaktionskammer, die an einer Begrenzungswand ihrer Innenhöhlung ein Strömungshindernis aufweist,
- Fig. 2: einen vergrößerten Ausschnitt von Fig. 1, der eine mit einem Strömungshindernis versehene Begrenzungswand der Innenhöhlung zeigt, die einen Aufnahmebereich für biologische Zellen aufweist,
- Fig. 3: eine grafische Darstellung der Strömungsgeschwindigkeit eines in der Innenhöhlung der in Fig. 1 gezeigten Messkammer befindlichen, von einer Einlassöffnung zu- und einer Auslassöffnung geförderten Fluids in einer Ebene, die einen Abstand von 51,2 µm zum Boden der Innenhöhlung der Reaktionskammer aufweist wobei in der Legende die den Grauschattierungen zugeordnete Geschwindigkeit in mm/s angegeben ist,
- Fig.4: eine grafische Darstellung ähnlich Fig. 3 für eine aus dem Stand der Technik bekannte Reaktionskammer, die an der Begrenzungswand kein Strömungshindernis aufweist,
- Fig.5: eine Teilansicht der das Strömungshindernis aufweisenden Begrenzungswand,
- Fig.6: eine Aufsicht auf die das Strömungshindernis aufweisende Begrenzungswand, und
- Fig. 7: eine Aufsicht auf einen Sensorchip, der in die Begrenzungswand integriert ist.

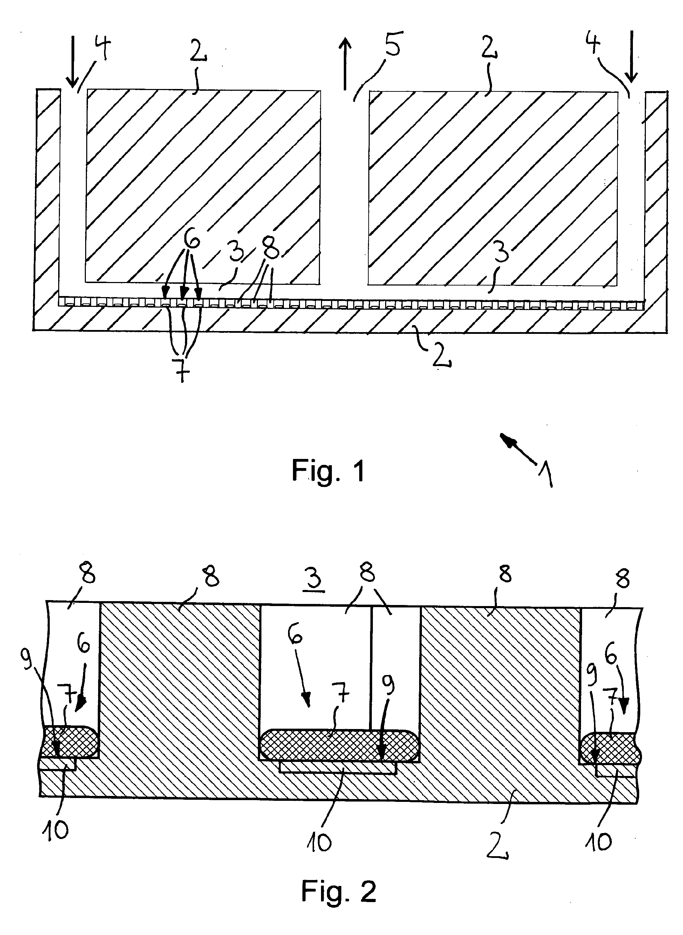

Eine in Fig. 1 im Ganzen mit 1 bezeichnete Reaktionskammer weist eine von Begrenzungswänden 2 umschlossene Innenhöhlung 3 mit einer etwa ringförmigen Einlassöffnung 4 und einer etwa mittig dazu angeordneten Auslassöffnung 5 für ein Fluid, wie z.B. eine Nährflüssigkeit für biologische Zellen, auf Die Einlassöffnung 4 ist über eine in der Zeichnung nicht näher dargestellte Fördereinrichtung, wie z.B. eine Pumpe oder eine Gefällestrecke, mit einem Fluid-Reservior verbunden. Mit Hilfe der Fördereinrichtung wird intermittierend frisches Fluid aus dem Fluid-Reservior der Innenhöhlung 3 zugeführt wobei in der Innenhöhlung eine Fluid-Strömung auftritt, die von der Einlassöffnung 4 etwa radial zu der Auslassöffnung 5 verläuft.

An einer den Boden der Innenhöhlung 3 bildenden Begrenzungswand 2 ist ein Aufnahmebereich 6 für strömungsempfindliche Reaktionszentren 7 angeordnet. In Fig. 1 und 2 sind als Reaktionszentren 7 lebende biologische Zellen vorgesehen, die an der Begrenzungswand 2 immobilisiert sind. Zwischen den Reaktionszentren 7 und dem Fluid findet ein Stoffaustausch statt, bei dem die Reaktionszentren 7 Nährstoffe aus dem Fluid aufnehmen und Ausscheidungsprodukte, die beim Stoffwechsel der Zellen entstehen, an das Fluid abgeben.

Die Begrenzungswand 2, an der die Reaktionszentren 7 anhaften, weist ein Strömungshindernis für die Fluid-Strömung auf, das eine Vielzahl von säulenartigen Vorsprüngen 8 hat, die derart zu den Reaktionszentren 7 benachbart sind, dass die Geschwindigkeit der Fluid-Strömung in dem Aufnahmebereich 6 für die Reaktionszentren 7 geringer ist als an einer Stelle der Innenhöhlung 3, die außerhalb des Aufiahmebereichs angeordnet und weiter von der Begrenzungswand 2 entfernt ist als der Aufnahmebereich. Die Säulen sind mit ihrer Längsachse jeweils etwa normal zur Erstreckungsebene der Begrenzungswand 2 angeordnet. Zwischen den Vorsprüngen 8 sind in einer Fluid-Schicht, die zwischen dem Grund 9 der Begrenzungswand 2 und den am weitesten vorstehenden Stellen der Vorsprünge 8 angeordnet ist, und die etwa parallel zur Begrenzungswand 2 verläuft Durchlässe gebildet, d.h. die zwischen den Vorsprüngen 8 befindlichen Zwischenräume sind seitlich miteinander verbunden. Dies wird dadurch erreicht dass die einzelnen Vorsprünge 8 an ihrem gesamten Außenumfang mit dem Fluid in Kontakt stehen, so dass alle Stellen des Aufiahmebereichs miteinander verbunden sind.

In Fig. 2 ist erkennbar, dass die Reaktionszentren 7 zwischen den Vorsprüngen 8 direkt am Grund 9 der Begrenzungswand 2 angeordnet sind, und dass die Höhe der Vorsprünge 8, d.h. die Abmessung, welche die Vorsprünge 8 in einer normal zur Erstreckungsebene der Begrenzungswand 2 verlaufenden Richtung aufweisen, größer ist als die entsprechende Abmessung der Reaktionszentren 7. Bei dem in Fig. 1 und 2 gezeigten Ausführungsbeispiel beträgt die Höhe der Reaktionszentren 7 etwa das Fünffache der Höhe der Reaktionszentren 7. Die Vorsprünge 8 überragen also die Reaktionszentren 7 deutlich.

Durch die Vorsprünge 8 ergibt sich in einer direkt zum Grund 9 der Begrenzungswand benachbarten, parallel zur Erstreckungsebene der Begrenzungswand 2 verlaufenden Fluid-Schicht parallel zur Erstreckungsebene ein wesentlich größerer Strömungswiderstand als in einer parallel zu dieser Schicht angeordneten, von den Vorsprüngen 8 beabstandeten Fluid-Schicht.

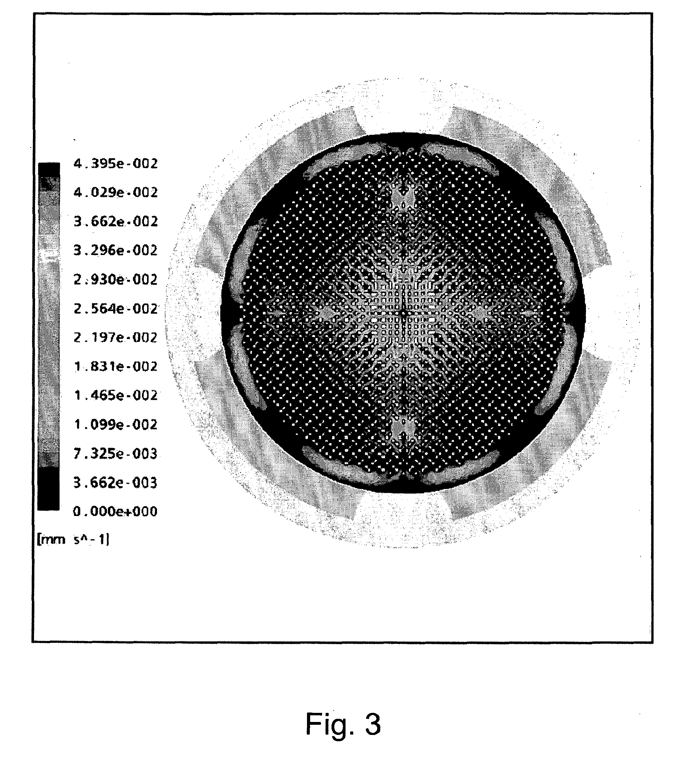

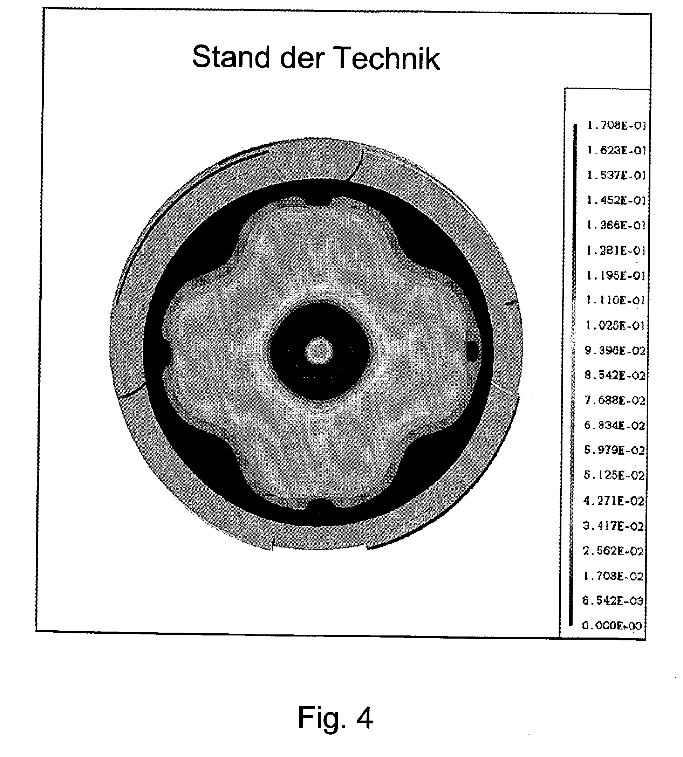

In Fig. 3 ist erkennbar, dass die Strömungsgeschwindigkeit des Fluids in einer Ebene, die in einem Abstand von 51,2 µm zum Grund der Begrenzungswand 2 und somit etwa in Höhe der am weitesten vorstehenden Stellen der Vorsprünge 8 verläuft, relativ gering und über die Fläche dieser Ebene weitgehend homogen ist. Im Vergleich dazu ist in Fig. 4 die Geschwindigkeitsverteilung für eine entsprechende Reaktionskammer dargestellt, die kein Strömungshindernis aufweist. Deutlich ist erkennbar, dass vor allem in einem benachbart zu der Auslassöffnung 5 angeordneten Bereich wesentlich größere Strömungsgeschwindigkeiten auftreten als bei der das Strömungshindernis aufweisenden Reaktionskammer. Dabei ist die Geschwindigkeit in Fig. 4 um mehr als eine Zehnerpotenz größer als in Fig. 3.

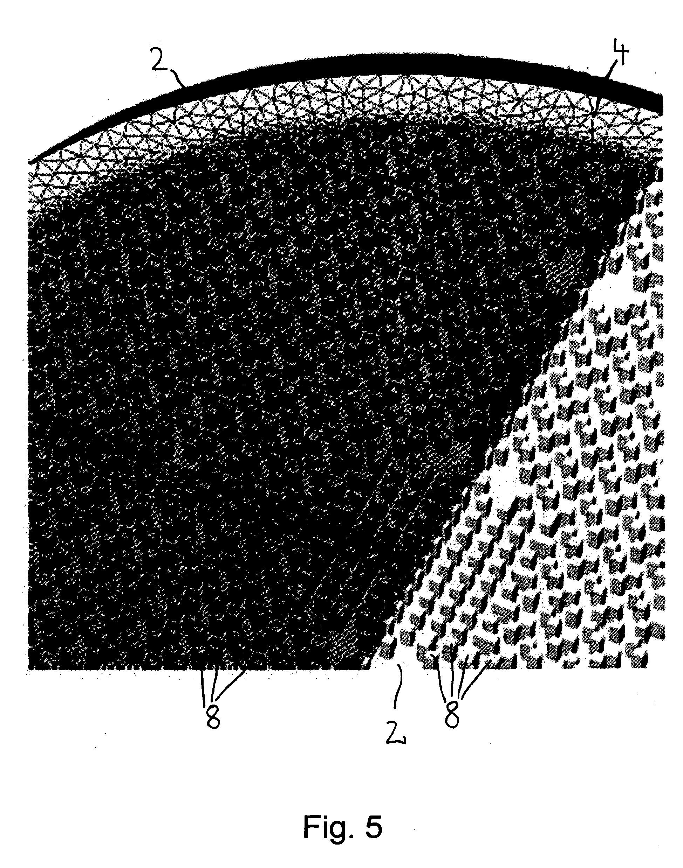

In Fig. 5 ist erkennbar, dass die Vorsprünge 8 unterschiedliche Querschnittsabmessungen haben und dass der Querschnitt der Vorsprünge 8 jeweils etwa rechteckförmig ist. Die Höhe der Vorsprünge ist bei allen Vorsprüngen etwa gleich. Der Abstand zwischen den einander zugewandten Wandflächen zueinander benachbart nebeneinander befindlicher Vorsprünge 2 entspricht etwa der Höhe der Vorsprünge 2.

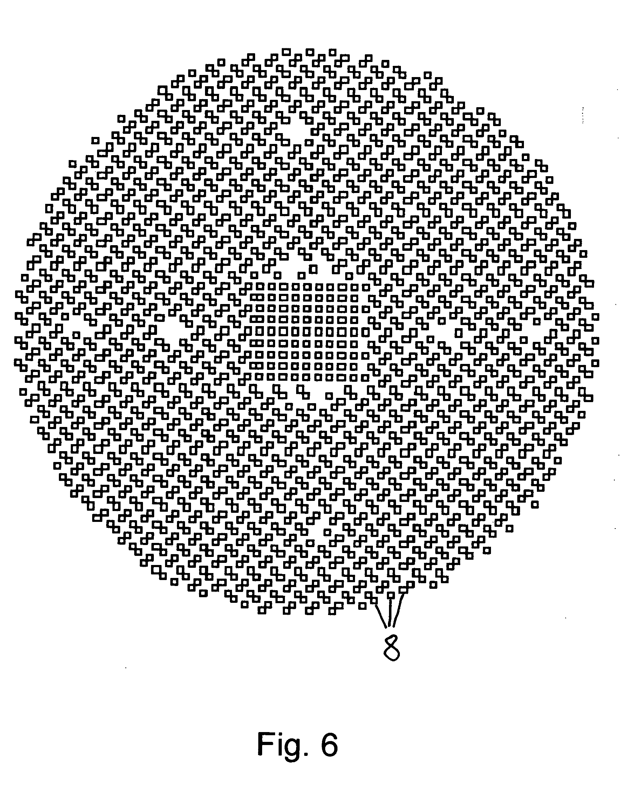

In Fig. 5 und 6 ist ferner erkennbar, dass in einem etwa quadratischen zentralen Bereich des Strömungshindernisses die Vorsprünge 2 etwa matrixförmig mit mehreren parallel zueinander verlaufende Reihen und Spalten angeordnet sind. Innerhalb der Reihen und Spalten sind die Vorsprünge 2 jeweils durch den Aufnahmebereich 6 voneinander beabstandet. In Hauptströmungsrichtung des Fluids betrachtet sind die Vorsprünge 2 dieser Reihen bzw. Spalten auf Lücke zueinander versetzt. Die Vorsprünge 2 sind also so angeordnet, dass es zwischen den Vorsprüngen 2 keine gerade Verbindungslinie gibt, welche die Einlassöffnung mit der Auslassöffnung 5 verbindet.

In dem zentralen Bereich haben die Vorsprünge 2 einen etwa quadratischen Querschnitt. Am Rand des zentralen Bereichs sind einige Vorsprünge 2 vorgesehen, die einen rechteckigen Querschnitt haben, bei dem die eine Seite des Rechtecks etwa doppelt so lang ist wie die andere Seite. Diese Vorsprünge 2 sind mit ihren längeren Seiten in unterschiedliche Richtungen orientiert, die entweder in Richtung der Reihen oder in Richtung der Spalten verlaufen.

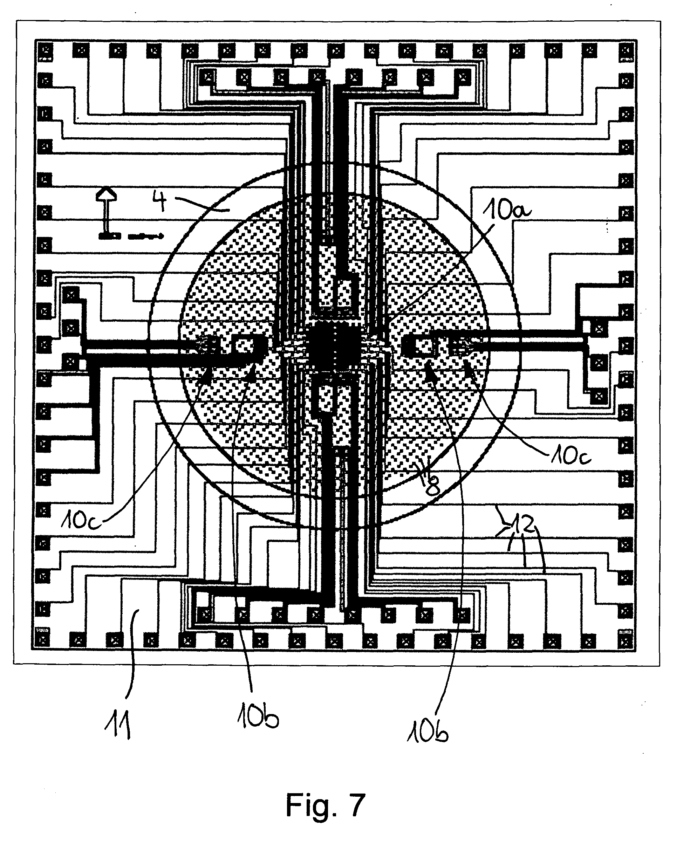

In den zwischen den Vorsprüngen befindlichen Lücken sind in dem zentralen Bereich erste Sensoren 10a in den Boden der Begrenzungswand 2 eingelassen, die auf einem Halbleiterchip 11 matrixförmig in mehreren Reihen und Spalten angeordnet sind (Fig. 7). Die Sensoren 10a können als Metall-Elektroden und/oder Zellpotential-FETs ausgestaltet sein. Zusätzlich sind auf dem Halbleiterchip 11 zweite Sensoren 10b angeordnet die als ionenselektive FETs ausgebildet sind. Dritte Sensoren 10c dienen zur Messung einer Gaskonzentration, wie z.B. der Sauerstoff konzentration. Mit Hilfe der Sensoren 10a, 10b, 10c können direkt an den Reaktionszentren 7 Messungen vorgenommen werden. Die Sensoren 10a, 10b, 10c sind über Leiterbahnen mit einer in der Zeichnung nicht näher dargestellten Auswerteeinrichtung verbunden.

## Patentansprüche

1. Reaktionskammer (1) mit einer Innenhöhlung (3), die mindestens eine Einlassöffnung (4) und wenigstens eine Auslassöffnung (5) für ein Fluid aufweist, mit einer Fördereinrichtung zum Erzeugen einer in Innenhöhlung (3) von der Einlassöffnung (4) zu der Auslassöffnung (5) verlaufenden Fluid-Strömung, wobei die Reaktionskammer (1) zumindest eine von der Fluid-Strömung durch Scherkräfte beaufschlagte Begrenzungswand (2) hat, an der wenigstens ein Aufnahmebereich (6) für ein strömungsempfindliches Reaktionszentrum (7), wie z.B. eine an der Begrenzungswand (2) immobiliserte biologische Zelle, vorgesehen ist, das dazu geeignet ist, einen in dem Fluid enthaltenen Stoff aufzunehmen und/oder einen Stoff an das Fluid abzugeben, **dadurch gekennzeichnet dass** die Begrenzungswand (2) mindestens ein Strömungshindernis für die Fluid-Strömung aufweist, das derart zu dem Aufnahmebereich (6) benachbart ist, dass die Geschwindigkeit der Fluid-Strömung in dem Aufnahmebereich (6) geringer ist als außerhalb des Aufnahmebereichs (6).

2. Reaktionskammer (1) nach Anspruch 1, **dadurch gekennzeichnet, dass** das Strömungshindernis eine Vielzahl von an der Begrenzungswand (2) vorstehenden Vorsprüngen (8) aufweist, die in Hauptströmungsrichtung der Fluid-Strömung und/oder quer dazu voneinander beabstandet sind und den Aufnahmebereich (6) zwischen sich einschließen.

3. Reaktionskammer (1) nach Anspruch 1 oder 2, **dadurch gekennzeichnet, dass** die Vorsprünge (8) unterschiedliche Querschnittsabmessungen haben.

4. Reaktionskammer (1) nach einem der Ansprüche 1 bis 3, **dadurch gekennzeichnet, dass** das Strömungshindernis mindestens zwei sich quer zur Hauptströmungsrichtung der Fluid-Strömung erstreckende Reihen mit voneinander beabstandeten Vorsprüngen (8) aufweist, dass die Reihen in Hauptströmungsrichtung voneinander beabstandet sind, und dass die Vorsprünge (8) einer ersten Reihe auf Lücke zu den Vorsprüngen (8) einer zweiten Reihe versetzt sind.

5. Reaktionskammer (1) nach einem der Ansprüche 1 bis 4, **dadurch gekennzeichnet, dass** die Abmessungen, welche die Vorsprünge (8) quer zur Erstreckungsebene der Begrenzungswand aufweisen größer sind als der lichte Abstand zwischen den Vorsprüngen (8).

6. Reaktionskammer (1) nach einem der Ansprüche 1 bis 5, **dadurch gekennzeichnet, dass** die Abmessungen, welche die Vorsprünge (8) quer zur Erstreckungsebene der Begrenzungswand aufweisen und/oder der lichte Abstand zwischen den Vorsprüngen (8) zwischen 10 und 200 µm, insbesondere zwischen 20 und 100 µm, gegebenenfalls zwischen 30 und 70 µm und bevorzugt zwischen 40 und 60 µm betragen (beträgt).

7. Reaktionskammer (1) nach einem der Ansprüche 1 bis 6, **dadurch gekennzeichnet, dass** der Fluid-Strömung zugewandte Seitenflächen der Vorsprünge (8) eine konkave Form aufweisen.

8. Reaktionskammer (1) nach einem der Ansprüche 1 bis 7, **dadurch gekennzeichnet, dass** die Vorsprünge als Säulen ausgestaltet sind, die mit ihrer Längserstreckung vorzugsweise etwa normal zur Erstreckungsebene der Begrenzungswand angeordnet sind.

9. Reaktionskammer (1) nach einem der Ansprüche 1 bis 8, **dadurch gekennzeichnet, dass** die Säulen einen eckigen, vorzugsweise rechteckigen und/oder quadratischen Querschnitt aufweisen.

10. Reaktionskammer (1) nach einem der Ansprüche 1 bis 9, **dadurch gekennzeichnet, dass** mindestens zwei Säulen mit rechteckigem oder länglichem Querschnitt mit ihrer Breitseite in unterschiedliche, vorzugsweise rechtwinklig zueinander verlaufende Richtungen weisen.

11. Reaktionskammer (1) nach einem der Ansprüche 1 bis 10, **dadurch gekennzeichnet dass** mehrere vorzugsweise auf einer Kreislinie angeordnete Einlassöffnungen (4) und/oder eine ringförmige Einlassöffnung (4) um eine zentrale Auslassöffnung (5) herum angeordnet sind (ist), und dass der Aufnahmebereich (6) und das Strömungshindernis zwischen den Einlassöffnungen (4) (der Einlassöffnung (4)) und der Auslassöffnung (5) vorgesehen sind und diese vorzugsweise ringförmig umgrenzen.

12. Reaktionskammer (1) nach einem der Ansprüche 1 bis 11, **dadurch gekennzeichnet, dass** mehrere vorzugsweise auf einer Kreislinie angeordnete Auslassöffnungen (5) und/oder eine ringförmige Auslassöffnung (5) um eine zentrale Einlassöffnung (4) herum angeordnet sind (ist), und dass der Aufnahmebereich (6) und das Strömungshindernis zwischen den Auslassöffnungen (5) (der Auslassöffnung (5)) und der Einlassöffnung (4) vorgesehen sind und diese vorzugsweise ringförmig umgrenzen.

13. Reaktionskammer (1) nach einem der Ansprüche 1 bis 12, **dadurch gekennzeichnet, dass** in die Begrenzungswand (2) mindestens ein Sensor (1 0a, 10b. 10c) zur Durchführung von Messungen an dem Reaktionszentrum (7) integriert ist, und dass vorzugsweise mehrere Sensoren (7) zur Messung unterschiedlicher Parameter, insbesondere des Aktionspotentials und/oder mindestens eines metabolischen Parameters einer biologischen Zelle nebeneinander angeordnet sind, vorzugsweise matrixförmig in mehreren Reihen und Spalten.

14. Reaktionskammer (1) nach einem der Ansprüche 1 bis 13, **dadurch gekennzeichnet, dass** die Säulen zwischen den Sensoren (10a, 10b, 10c) matrixförmig in mehreren Reihen und Spalten nebeneinander angeordnet sind.
